# EUROPÄISCHE PATENTANMELDUNG

(11) **EP 2 826 384 A1**
(43) Veröffentlichungstag der Anmeldung: **21.01.2015**
(21) Anmeldenummer: 13176661.0
(22) Anmeldetag: 16.07.2013
(51) Int. Cl.: A23K 1/00, A23K 1/16, A23K 1/18, C12N 1/12

(54) **Verfahren zur Trocknung von Biomasse**

(71) Anmelder: Evonik Industries AG, 45128 Essen (DE)
(72) Erfinder: Rudinger, Nicolas, Dr., 40215 Düsseldorf (DE); Rabe, Christian, 63762 Großostheim (DE); Blümke, Wilfried, Dr., 61137 Schöneck (DE)

(57) **Zusammenfassung**

Erfindungsgemäß wurde gefunden, dass eine einen oxidationsempfindlichen Wertstoff enthaltene Biomasse besonders schonend durch ein Verfahren getrocknet werden kann, in welchem das Trocknungsgas in Kreisgasfahrweise über die zu trocknende Biomasse geleitet wird.

## Beschreibung

Die vorliegende Erfindung betrifft ein Verfahren zur schonenden Trocknung einer Biomasse, insbesondere einer Biomasse, die einen oxidationsempfindlichen Wertstoff enthält, sowie die durch dieses Verfahren erhaltene Biomasse.

Die Bedeutung von mikrobiellen Zellen zur Herstellung von Wertstoffen ist dem Fachmann bekannt. Ein Beispiel für solche Wertstoffe stellen Nahrungsmittelkomponenten dar, insbesondere Lipide wie etwa polyungesättigte Fettsäuren. Für die Herstellung von solchen Wertstoffen spielen neben Bakterien und Hefen insbesondere auch weitere Pilze sowie Algen eine besondere Rolle.

Bestimmte Wertstoffe, insbesondere polyungesättigte Fettsäuren (PUFAs), stellen eine wichtige Komponente für die Ernährung von Mensch und Tier dar. Als Quelle für omega-3-Fettsäuren wurde anfangs vor allem Fisch verwendet. Später wurde entdeckt, dass bestimmte Mikroben heterotroph omega-3-Fettsäuren in großen Mengen produzieren, wobei die Fettsäureproduktion durch Wahl spezifischer Reaktionsparameter vorteilhaft beeinflusst werden kann. Die omega-3-Fettsäuren können anschließend aus den Zellen gewonnen werden oder aber die Zellen in Form von Biomasse direkt in Futter- oder Nahrungsmitteln eingesetzt werden.

Ein Problem bei der Verwendung und Verarbeitung von zahlreichen Wertstoffen, insbesondere von polyungesättigten Fettsäuren, stellt ihre Instabilität gegenüber oxidativem Abbau dar: Sobald der Wertstoff aus den Zellen isoliert wird, ist die Gefahr des oxidativen Abbaus stark erhöht, da der Schutz durch die umgebende Zellmembran nicht mehr gegeben ist.

Erfindungsgemäß hat sich jedoch herausgestellt, dass bereits durch schonende Trocknung der den Wertstoff enthaltenden Biomasse eine deutliche Verbesserung der Eigenschaften des enthaltenen Wertstoffs erzielt werden kann, wobei sich ein Trocknungsverfahren, bei welchem Gas in Kreisgasfahrweise über die Biomasse geleitet wird, als besonders vorteilhaft herausgestellt hat.

Die Aufgabe der vorliegenden Erfindung kann daher darin gesehen werden, einen schonenden Weg zur Trocknung von Biomasse, die einen oxidationsempfindlichen Wertstoff enthält, aufzuzeigen.

Gelöst wird die erfindungsgemäße Aufgabe durch ein Verfahren zur Trocknung einer Biomasse, die einen oxidationsempfindlichen Wertstoff enthält, **dadurch gekennzeichnet, dass** das Verfahren einen Trocknungsschritt umfasst, in welchem Gas in Kreisgasfahrweise über die Biomasse geleitet wird.

"Kreisgasfahrweise" bedeutet, dass das zur Trocknung verwendete Gas zirkulierend über die Biomasse geleitet wird.

Bei dem Verfahrensschritt, der in der Überleitung von Gas in Kreisgasfahrweise über die Biomasse besteht, handelt es sich vorzugsweise um ein thermisches Verfahren. D.h. das eingesetzte Gas hat vorzugsweise eine Temperatur oberhalb der Sättigungstemperatur des zu verdampfenden Lösungsmittels.

Bei dem eingesetzten Gas handelt es sich vorzugsweise um Luft mit reduziertem Gehalt an Sauerstoff.

Das in Kreisgasfahrweise geführte Gas weist vorzugsweise einen Sauerstoffgehalt von weniger als 20 Gew.-%, vorzugsweise weniger als 15 Gew.-%, insbesondere von 5 bis 13 Gew.-%, auf.

Das Gas wir vorzugsweise dadurch erzeugt, das Luft über einen Brenner geleitet und auf diese Weise erhitzt wird. Zugleich wird hierdurch der Sauerstoffgehalt der Luft auf unter 20 Gew.-%, vorzugsweise auf weniger als 15 Gew.-%, insbesondere auf 5 bis 13 Gew.-% reduziert. Das Gas wird stets auf gleiche Weise nachreguliert, um einen konstanten Gasstrom mit vermindertem Sauerstoffgehalt zu erzeugen.

Bei dem Trocknungsschritt, in welchem das Gas in Kreisgasfahrweise zur Trocknung der Biomasse verwendet wird, wird die Trocknung vorzugsweise in einem Wirbelschichtverfahren durchgeführt. Besonders bevorzugt wird in diesem Trocknungsschritt die Biomasse unmittelbar in ein Granulat umgewandelt, so dass es sich um ein Sprühgranulationsverfahren handelt.

Ein besonderer Vorteil dieses Verfahrens besteht darin, dass in der Fermentationsbrühe enthaltene Biomasse in einem Schritt getrocknet und granuliert werden kann und somit nur ein Verfahrensschritt von der Biomasse enthaltenden Fermentationsbrühe bis zum fertigen Produkt erforderlich ist.

Ein weiterer Vorteil dieses Verfahrens besteht darin, dass das Wirbelschichtverfahren kontinuierlich und stationär betrieben werden kann: Biomasse enthaltende Fermentationsbrühe kann kontinuierlich eingesprüht werden und das fertige Produkt kann kontinuierlich ausgetragen werden. Es ist hierbei vorzugsweise auch die Herstellung eines spezifikationsgerechten Produkts ohne Zusatz von Zuschlagstoffen möglich.

Die Wirbelschicht hat hierbei vorzugsweise eine Temperatur von 45 bis 95 °C, insbesondere 45 bis 75 °C, besonders bevorzugt 50 bis 60 °C. Die Luft wird entsprechend so stark erhitzt, dass sich in der Wirbelschicht eine Temperatur von 45 bis 95 °C, insbesondere 45 bis 75 °C, besonders bevorzugt 50 bis 60 °C, einstellt.

Die erfindungsgemäß einzusetzende Biomasse umfasst Zellen, kann jedoch darüber hinaus weitere Bestandteile umfassen. Bei der Biomasse handelt es sich vorzugsweise um das Produkt eines fermentativen Kultivierungsverfahrens. Die Biomasse kann entsprechend neben den Zellen insbesondere noch Bestandteile des Fermentationsmediums enthalten. Bei diesen Bestandteilen kann es sich insbesondere um Salze, Antischaummittel und nicht umgesetzte Kohlenstoffquelle und/oder Stickstoffquelle handeln. Der Zellgehalt in dieser Biomasse beträgt vorzugsweise mindestens 70 Gew.-%, vorzugsweise mindestens 75 Gew.-%. Der Zellgehalt in der Biomasse kann gegebenenfalls vor Durchführung der Trocknung durch entsprechende Waschschritte beispielsweise auf mindestens 80 oder mindestens 90 Gew.-% erhöht werden. Die im Fermentationsverfahren erhaltene Biomasse kann jedoch auch direkt in dem Trocknungsverfahren eingesetzt werden.

Bei den in der Biomasse enthaltenen Zellen kann es sich um Zellen handeln, die bereits natürlicherweise Wertstoffe, vorzugsweise Lipide, insbesondere PUFAs, produzieren, es kann sich jedoch auch um Zellen handeln, die durch entsprechende gentechnische Verfahren dazu in die Lage versetzt wurden, Lipide, insbesondere PUFAs, zu produzieren. Die Produktion kann hierbei autotroph, mixotroph oder heterotroph erfolgen.

Bevorzugt umfasst die Biomasse Zellen, die Lipide, insbesondere PUFAs, heterotroph produzieren. Es handelt sich bei den Zellen erfindungsgemäß vorzugsweise um Algen, Pilze, insbesondere Hefen, oder Protisten, es kommen jedoch etwa auch Zellen von öl-produzierenden Pflanzen in Betracht. Besonders bevorzugt handelt es sich um Zellen mikrobieller Algen oder Pilze.

Als Zellen von öl-produzierenden Pflanzen kommen insbesondere die Samen von Soja, Flachs, Raps, Mais, Baumwolle, Distel und Sonnenblume in Betracht.

Als Zellen von öl-produzierenden Hefen kommen insbesondere Stämme von Yarrowia, Candida, Rhodotorula, Rhodosporidium, Cryptococcus, Trichosporon und Lipomyces in Betracht.

Erfindungsgemäß umfasst die Biomasse vorzugsweise Zellen des Taxons Labyrinthulomycetes (Labyrinthulea, Netzschleimpilze, Schleimnetze), insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium und Ulkenia. Besonders bevorzugt umfasst die Biomasse Zellen der Gattungen Thraustochytrium, Schizochytrium oder Ulkenia, vor allem solche der Gattung Thraustochytrium.

Bei dem oxidationsempfindlichen Wertstoff handelt es sich vorzugsweise um ein oxidationsempfindliches Lipid, insbesondere um eine ungesättigte Fettsäure, besonders bevorzugt um eine polyungesättigte Fettsäure (PUFA) oder hochungesättigte Fettsäure (HUFA).

Die in der Biomasse enthaltenen Zellen zeichnen sich vorzugsweise dadurch aus, dass sie einen Wertstoff-Gehalt, vorzugsweise Lipid-Gehalt, besonders bevorzugt PUFA-Gehalt, von mindestens 20 Gew.-%, vorzugsweise mindestens 30 Gew.-%, insbesondere mindestens 40 Gew.-%, jeweils bezogen auf die Zelltrockenmasse, aufweisen.

In einer bevorzugten Ausführungsform liegt hierbei ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Weiterhin umfassen die in der Zelle enthaltenen Lipide vorzugsweise polyungesättigte Fettsäuren (PUFAs), wobei vorzugsweise mindestens 10 Gew.-%, insbesondere mindestens 20 Gew.-%, besonders bevorzugt 20 bis 60 Gew.%, insbesondere 20 bis 40 Gew.-%, der in der Zelle enthaltenen Fettsäuren PUFAs darstellen.

Unter polyungesättigten Fettsäuren (PUFAs) werden erfindungsgemäß Fettsäuren verstanden, die mindestens zwei, insbesondere mindestens drei, C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Verfahren zur Herstellung der Biomasse, insbesondere solcher Biomasse, die Lipide, insbesondere PUFAs, enthaltende Zellen, insbesondere der Ordnung Thraustochytriales, umfasst, sind im Stand der Technik ausführlich beschrieben. Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden (WO 01/54510). Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon (WO 01/54510).

Die Fermentation der Zellen erfolgt vorzugsweise in einem Medium mit niedriger Salinität, isnbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Die Fermentation der Zellen erfolgt vorzugsweise bei einem pH-Wert von 5 bis 11, insbesondere 6 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Nach Beendigung der Fermentation erfolgt die Ernte der Biomasse. Vorzugsweise erfolgt nach der Ernte der Biomasse oder gegebenenfalls auch schon kurz vor der Ernte der Biomasse eine Pasteurisierung der Zellen, um die Zellen abzutöten und Enzyme, die den Abbau der Lipide befördern könnten, zu inaktivieren. Die Pasteurisierung erfolgt vorzugsweise durch Erhitzen der Biomasse auf eine Temperatur von 50 bis 121 °C für eine Dauer von 5 bis 60 Minuten.

Ebenso erfolgt nach der Ernte der Biomasse oder gegebenenfalls auch schon kurz vor der Ernte der Biomasse vorzugsweise die Zugabe von Antioxidantien, um den in der Biomasse enthaltenen Wertstoff vor oxidativem Abbau zu schützen. Bevorzugte Antioxidantien stellen hierbei BHT, BHA, TBHA, Ethoxyquin, beta-Carotin, Vitamin E und Vitamin C dar. Das Antioxidans wird, sofern eingesetzt, vorzugsweise in einer Menge von 0,01 bis 2 Gew.-% hinzugegeben.

In einem ersten Schritt kann nun bereits ein Teil des Fermentationsmediums von der Biomasse abgetrennt und damit der Feststoffanteil erhöht werden. Dies kann insbesondere durch Zentrifugation, Filtration, insbesondere Ultra- oder Mikrofiltration, Dekantieren und/oder Lösungsmittelverdampfung erfolgen. Die Lösungsmittelverdampfung erfolgt hierbei vorzugsweise unter Einsatz eines Rotationsverdampfers, eines Dünnschichtverdampfers oder eines Fallfilmverdampfers in einem einstufigen oder mehrstufigen Prozess. Alternativ zur Lösungsmittelverdampfung kommt beispielsweise auch die Umkehrosmose zwecks Einengung der Fermentationsbrühe in Betracht.

In diesem ersten optionalen, aber bevorzugt durchgeführten, Schritt erfolgt vorzugsweise eine Aufkonzentrierung des Fermentationsmediums auf einen Feststoffgehalt von mindestens 10 oder 15 Gew.-%, vorzugsweise von mindestens 20 oder 25 Gew.-%, insbesondere 10 bis 50 oder 15 bis 45 Gew.-%, besonders bevorzugt 20 bis 40 Gew.-% oder 25 bis 40 Gew.-%.

D.h. die in einem erfindungsgemäßen Verfahren zu trocknende Biomasse liegt vorzugsweise in Form einer Suspension mit dem zuvor angegebenen Feststoffanteil vor, wobei es sich bei dem flüssigen Bestandteil der Suspension vorzugsweise um Fermentationsbrühe oder eingeengte Fermentationsbrühe handelt.

Nach Einengung bzw. teilweiser Abtrennung der Fermentationsbrühe erfolgt die erfindungsgemäße Trocknung der Biomasse durch Überleitung von vorzugsweise erwärmtem Gas über die Biomasse bzw. Biomasse-Suspension in der Kreisgasfahrweise.

Alternativ kann die Biomasse auch direkt nach der Ernte durch ein Kreisgasverfahren getrocknet werden, insbesondere wenn die erhaltene Fermentationsbrühe bereits einen hohen Feststoffgehalt, vorzugsweise wie zuvor angegeben, aufweist.

Wie zuvor gesagt wird zur Trocknung vorzugsweise Luft verwendet, deren Sauerstoffkonzentration vorzugsweise wie zuvor angegeben reduziert ist.

Durch die Trocknung der Biomasse in der Kreisgasfahrweise wird die Suspension vorzugsweise auf eine Restfeuchte von maximal 10 Gew.-%, insbesondere 0 bis 10 Gew.-%, besonders bevorzugt maximal 8 Gew.-%, insbesondere 0,5 bis 8 Gew.-%, vor allem maximal 6 oder 5 Gew.-%, insbesondere 1 bis 6 oder 1 bis 5 Gew.-%, getrocknet.

Wie zuvor bereits ausgeführt erfolgt die erfindungsgemäße Trocknung der Biomasse vorzugsweise in einem Wirbelschichtgranulationsverfahren. Die Biomasse enthaltende Fermentationsbrühe wird hierzu in die Wirbelschicht-Trocknungsgranulationsanlage eingesprüht. Eine erfindungsgemäß vorzugsweise einsetzbare Trocknungsgranulationsanlage ist in Fig. 1 von EP 0809940 schematisch dargestellt.

In die Wirbelschicht-Trocknungsgranulationsanlage wird das Trocknungsgas von unten eingeleitet. Der Großteil der Feuchtigkeit der eingesprühten Fermentationsbrühe verdampft und das entstehende Granulat wird durch den Gasstrom des Trocknungsgases in der Schwebe gehalten. In diesem Zustand sind die Partikel voneinander getrennt und so beim Einsprühen von weiterer Flüssigkeit in das Bett für die Sprühtropfen frei zugänglich. Außerdem ist in diesem Zustand der Wärme- und Stoffaustausch zwischen den Feststoffpartikeln und dem Gasstrom intensiv. Produktpartikel mit der gewünschten Größe werden kontinuierlich in einem klassierenden Abzug aus der Wirbelschicht abgezogen.

Die Wirbelschicht oder das Bett aus Partikeln, welches zu Beginn der Wirbelschicht-Granulationstrocknung vorhanden sein muss, besteht vorzugsweise aus getrockneten Partikeln der zur Trocknung eingesetzten Biomasse, beispielsweise aus einer Charge eines vorherigen Laufs. Es ist jedoch genauso gut möglich, einen anderen Stoff als Wirbelschicht zur Initiierung der Wirbelschicht-Granulationstrocknung einzusetzen.

Ein besonderer Vorteil des Wirbelschichtgranulationsverfahrens ist, dass das Verfahren kontinuierlich betrieben werden kann und in einem Schritt die Biomasse enthaltende Fermentationsbrühe in ein Produkt mit gewünschter Korngröße überführt werden kann.

Die erzeugten Partikel haben außerdem eine ausgezeichnete Festigkeit und weisen durch ihre im Wesentlichen runde Form bedingt sehr gute Schütt- und Fließeigenschaften auf. Die Partikel haben außerdem eine geringe Restfeuchte.

Durch die erfindungsgemäße Trocknung wird vorzugweise ein rieselfähiges, feinteiliges oder grobkörniges Produkt, vorzugsweise Granulat, erhalten. Gegebenenfalls kann aus dem erhaltenen Granulat durch Sieben oder Staubabtrennung ein Produkt mit der gewünschten Korngröße erhalten werden.

Sofern ein rieselfähiges feinteiliges Pulver erhalten wurde, kann dieses gegebenenfalls durch geeignete Kompaktier- oder Granulierverfahren in ein grobkörniges, gut rieselfähiges, lagerbares und weitgehend staubfreies Produkt überführt werden.

Bei der Granulation oder Kompaktierung können gegebenenfalls übliche organische oder anorganische Hilfsstoffe beziehungsweise Träger wie Stärke, Gelatine, Cellulosederivate oder ähnliche Stoffe, die überlicherweise in der Lebensmittel- oder Futterverarbeitung als Binde-, Gelier- oder Verdickungsmittel Verwendung finden, eingesetzt werden.

Unter "rieselfähig" ist erfindungsgemäß ein Pulver zu verstehen, das aus einer Serie von Glasauslaufgefäßen mit verschieden großen Auslassöffnungen mindestens aus dem Gefäß mit der Öffnung 5 Millimeter ungehindert auslaufen kann (Klein: Seifen, Öle, Fette, Wachse 94, 12 (1968)).

Unter "feinteilig" ist erfindungsgemäß ein Pulver mit einem überwiegenden Anteil (> 50 %) einer Korngröße von 20 bis 500 Mikrometer Durchmesser zu verstehen.

Unter "grobkörnig" ist erfindungsgemäß ein Pulver mit einem überwiegenden Anteil (>50 %) einer Korngröße von 500 bis 2500 Mikrometer Durchmesser zu verstehen.

Unter "staubfrei" ist erfindungsgemäß ein Pulver zu verstehen, das lediglich geringe Anteile (< 5 %) an Korngrößen unter 100 Mikrometer enthält.

Die Bestimmung der Korn- bzw. Teilchengrößen erfolgt erfindungsgemäß vorzugsweise durch Methoden der Laserbeugungsspektrometrie. Die anzuwendenden Methoden sind im Lehrbuch "Teilchengrößenmessung in der Laborpraxis" von R.H. Müller und R. Schuhmann, Wissenschaftliche Verlagsgesellschaft Stuttgart (1996) sowie im Lehrbuch "Introduction to Particle Technology" von M. Rhodes, Verlag Wiley & Sons (1998) beschrieben. Sofern unterschiedliche Methoden anwendbar sind, wird bevorzugt die zuerst genannte anwendbare Methode aus dem Lehrbuch von R.H. Müller und R. Schuhmann zur Teilchengrößenmessung angewendet.

Die erfindungsgemäß erhaltenen Produkte besitzen vorzugsweise einen Anteil von mindestens 80 Gew.-%, insbesondere mindestens 90 Gew.-%, besonders bevorzugt mindestens 95 Gew.-%, vor allem mindestens 97 Gew.-%, an Partikeln mit einer Korngröße von 100 bis 3500 Mikrometern, vorzugsweise 1000 bis 3000 Mikrometern, vor allem 1500 bis 2500 Mikrometern.

Aufgrund des Herstellverfahrens sind vorzugsweise mindestens 50 Gew.-%, insbesondere mindestens 70 Gew.-%, besonders bevorzugt mindestens 90 Gew.-%, vor allem im Wesentlichen alle Partikel im Wesentlichen sphärisch ausgebildet. Vor allem die im Wesentlichen sphärische Ausbildung der Partikel bedingt die hervorragenden Schütt- und Fließeigenschaften des erfindungsgemäßen Produkts.

Unter "im Wesentlichen sphärisch ausgebildet" ist erfindungsgemäß zu verstehen, dass der Durchmesser eines Partikels in alle Raumrichtungen im Wesentlichen gleich ist. Unter "im Wesentlichen gleich" ist hierbei zu verstehen, dass die Abweichung des Durchmessers eines Partikels in zwei beliebige Raumrichtungen maximal 20 %, vorzugsweise maximal 15 %, insbesondere maximal 10 %, besonders bevorzugt maximal 5 %, beträgt.

Der Anteil an Staub, d.h. Teilchen mit einer Korngröße von kleiner 100 Mikrometern, liegt vorzugsweise bei maximal 1 Gew.-%, besonders bevorzugt maximal 0,5 Gew.-%.

Das Schüttgewicht der erfindungsgemäßen Produkte liegt vorzugsweise bei 400 bis 800 kg/m3, besonders bevorzugt bei 450 bis 700 kg/m3.

Gegenstand der vorliegenden Erfindung ist somit auch eine partikuläre Biomasse, die durch ein erfindungsgemäßes Verfahren erhältlich ist sowie eine partikuläre Biomasse, die einen oxidationsempfindlichen Wertstoff enthält, mit den zuvor genannten Eigenschaften, insbesondere mit den zuvor genannten Eigenschaften hinsichtlich Partikelgröße und Partikelverteilung. Hinsichtlich bevorzugter Natur der Biomasse und bevorzugt enthaltenem Wertstoff wird auf das zuvor Ausgeführte verwiesen.

Die erfindungsgemäß erhaltene Biomasse kann auf unterschiedliche Weise verwendet werden. Nach der erfindungsgemäßen Trocknung der Biomasse erfolgt vorzugsweise eine Lagerung bzw. Verpackung der getrockneten Biomasse. Anschließend kann die Biomasse beispielsweise eingesetzt werden, um ein Futter- oder Nahrungsmittel herzustellen oder um den Wertstoff aus der Biomasse zu isolieren.

Ein Futter- oder Nahrungsmittel enthaltend eine erfindungsgemäße partikuläre Biomasse ist ein weiterer Gegenstand der vorliegenden Erfindung.

Weiterer Gegenstand der vorliegenden Erfindung ist daher auch ein Verfahren zur Isolierung des Wertstoffs aus einer erfindungsgemäßen partikulären Zusammensetzung.

Um die Bioverfügbarkeit in dem herzustellenden Futter- oder Nahrungsmittel zu erhöhen und/oder um die Isolierung des in der Biomasse enthaltenen Wertstoffs zu erleichtern, wird die partikuläre Biomasse vorzugsweise einem Zellaufschlussverfahren unterzogen. Die Isolierung des Wertstoffs kann alternativ auch direkt aus der partikulären Biomasse erfolgen, ohne diese vorher aufzuschließen.

Vor der Durchführung des Zellaufschlussverfahrens wird vorzugsweise ausgehend von der getrockneten Biomasse eine Zellsuspension hergestellt. Hierzu wird die getrocknete Biomasse mit Wasser oder einer wässrigen Lösung vermischt, um eine Zellsuspension mit einem Feuchtigkeitsgehalt von vorzugsweise mindestens 30 Gew.-%, insbesondere 30 bis 90 Gew.-%, besonders bevorzugt 40 bis 80 Gew.-%, vor allem 50 bis 75 Gew.-%, herzustellen.

Der Zellaufschluss kann anschließend unter Einsatz der dem Fachmann bekannten Zellaufschlussverfahren erfolgen, wie beispielsweise durch einen Schneckenextruder, eine Schlägermühle, eine Luftdüsenmühle oder durch Anwendung erhöhten Drucks, beispielsweise durch das sogenannte French-Press-Verfahren. Alternativ oder zusätzlich kann der Zellaufschluss durch den Einsatz von zellwandverdauenden Enzymen erfolgen.

Der Zellaufschluss wird erfindungsgemäß vorzugsweise unter Verwendung eines Rotor-Stator-Systems durchgeführt. Das Rotor-Stator-System basiert auf einem stationären Teil, der Stator genannt wird, und einem rotierenden Teil, dem Rotor. Der Rotor besitzt typischerweise eine Umfangsgeschwindigkeit von mindestens 5 m/s, beispielsweise 10 bis 30 m/s, die Spaltbreite zwischen Rotor und Stator kann beispielsweise 0,1 - 0,5 mm betragen. Zur Durchführung des Zellaufschlusses wird die Zell-Suspension in den Zwischenraum zwischen Stator und Rotor zugegeben. In diesem Spalt erfahren die Zellen eine Scherbeanspruchung und zusätzlich entstehen Turbulenzen. Diese beiden Faktoren bewirken den Zellaufschluss. In einer bevorzugten Ausführungsform erfolgt die Herstellung der Suspension im Rotor-Stator-System unter Verwendung eines feststoffmischenden Aufsatzes. Unter einem feststoffmischenden Aufsatz ist hierbei eine Vorrichtung zu verstehen, die das separate Einbringen von einerseits Feststoff und andererseits Wasser bzw. wässriger Lösung in das Rotor-Stator-System erlaubt. Die Suspension wird somit erst während des Zellaufschlusses bzw. unmittelbar vor dem Zellaufschluss durch Vermischung in dem feststoffmischenden Aufsatz hergestellt. Erfindungsgemäß wurde gefunden, dass unter Verwendung eines solchen feststoffmischenden Aufsatzes Suspensionen mit sehr hohen Feststoffgehalten dem Zellaufschluss unterzogen werden können, was mit Hinblick auf die nachfolgende Verarbeitung besonders vorteilhaft ist. Suspensionen, die unter Verwendung eines feststoffmischenden Aufsatzes in dem Rotor-Stator-System eingesetzt werden, weisen vorzugsweise einen Feststoffgehalt von 20-50 Gew.-%, besonders bevorzugt von 30-50 Gew.-%, auf.

Falls zur Herstellung der Zellsuspension eine wässrige Lösung eingesetzt wird, dann kann diese insbesondere weitere Nahrungsmittelkomponenten - wie etwa Vitamine oder Salze - enthalten.

Der Energieeintrag auf die Zellen, insbesondere unter Verwendung eines Rotor-Stator-Systems, beträgt erfindungsgemäß vorzugsweise maximal 50 kWh pro Tonne Suspension, insbesondere maximal 40, 35 oder 30 kWh pro Tonne Suspension, besonders bevorzugt maximal 25, 20 oder 15 kWh pro Tonne Suspension. Bevorzugte Bereiche stellen hierbei Energieeinträge von 0,1 - 50 kWh pro Tonne Suspension, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, dar.

Die "Zellaufschlussrate" des erfindungsgemäßen Verfahrens beträgt vorzugsweise mindestens 50 %, besonders bevorzugt mindestens 60, 70 oder 80 %, vor allem mindestens 85, 90 oder 95 %. Unter der "Zellaufschlussrate" ist die Anzahl der aufgeschlossenen Zellen nach Beendigung des Zellaufschlussverfahrens im Verhältnis zur Gesamtzahl der Zellen zu verstehen. Die Zellaufschlussrate kann beispielsweise visuell unter Verwendung eines Mikroskops bestimmt werden als das Verhältnis der Anzahl aufgeschlossener Zellen in Bezug zur Gesamtzahl der Zellen.

Um die Wertstoffe, insbesondere Lipide, gegen oxidativen Abbau zu stabilisieren, können in der Zellsuspension, die für den Zellaufschluss verwendet wird, zusätzlich Antioxidantien enthalten sein. Bevorzugte Antioxidantien stellen hierbei BHT, BHA, TBHA, Ethoxyquin, beta-Carotin, Vitamin E und Vitamin C dar. Das Antioxidans ist, sofern eingesetzt, vorzugsweise in einer Menge von 0,01 bis 2 Gew.-% enthalten. Die Antioxidantien werden hierbei in einer bevorzugten Ausführungsform bereits nach Beendigung der Fermentation dem Fermentationsmedium hinzugegeben.

Die Isolation des Wertstoffs aus der Biomasse kann sowohl ausgehend von der intakten getrockneten Biomasse als auch ausgehend von der aufgeschlossenen Biomasse erfolgen.

Zur Isolation des Wertstoffs aus der aufgeschlossenen Biomasse kann beispielsweise einfach eine mechanische Abtrennung der Zelltrümmer erfolgen, beispielsweise durch Dekantieren, Filtration oder Zentrifugation.

Die Isolation des Wertstoffs kann ansonsten sowohl aus der intakten als auch aus der aufgeschlossenen Biomasse beispielsweise durch Lösungsmittelextraktion erfolgen. Das Lösungsmittel kann nach der Abtrennung des Wertstoffs entsprechend wieder entfernt werden, beispielsweise durch Anlegen eines reduzierten Drucks. Alternativ kann die Isolation des Wertstoffs beispielsweise durch superkritische Flüssigextraktion erfolgen.

Als Lösungsmittel eingesetzt werden können die dem Fachmann bekannten Lösungsmittel, beispielsweise Chloroform, Ether, Hexan, Methylenchlorid oder Methanol. Die Abtrennung des Öls kann beispielsweise auch dadurch erfolgen, dass ein anderes Öl zur Extraktion des erfindungsgemäßen Öls verwendet wird.

Das Öl kann anschließend einer chemischen oder physikalischen Raffinierung unterzogen werden. Die Raffinierung kann das Degummieren, das Bleichen, das Filtern, das Desodorieren und/oder das Polieren des Rohöls umfassen. Anschließend können gegebenenfalls einzelne Ölbestandteile isoliert werden.

Sowohl die intakte als auch die aufgeschlossene Biomasse sowie die aus der Biomasse isolierten Wertstoffe können zur Herstellung eines Nahrungs- oder Futtermittels verwendet werden, bei welchem die Biomasse bzw. der Wertstoff vorzugsweise mit anderen Nahrungs- oder Futtermittelinhaltsstoffen vermischt werden und anschließend zu dem Nahrungs- oder Futtermittel verarbeitet werden.

Die Verarbeitung des Gemisches aus Biomasse und weiteren Nahrungs- oder Futtermittelinhaltsstoffen erfolgt in einer bevorzugten Ausführungsform durch ein Extrusionsverfahren, um verkaufsfertige Portionen des Nahrungs- oder Futtermittels zu erhalten. Alternativ kann beispielsweise auch ein Pelletierverfahren eingesetzt werden.

Im Extrusionsverfahren wird vorzugsweise ein Schnecken- oder Doppelschneckenextruder eingesetzt. Das Extrusionsverfahren wird vorzugsweise bei einer Temperatur von 80 - 220° C, insbesondere 100 - 190 °C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Bei einer erfindungsgemäß bevorzugten Art des Extrusionsverfahrens umfasst das Verfahren einen Kompaktierungs- und einen Kompressionsschritt.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken.

Die weiteren Nahrungs- oder Futtermittelinhaltsstoffe werden vor dem Vermischen mit den aufgeschlossenen Zellen - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Ein erfindungsgemäß bevorzugtes Verfahren zur Herstellung eines Nahrungs- oder Futtermittels umfasst daher die folgenden Schritte:
a) Herstellung einer Biomasse, vorzugsweise durch Fermentierung von Pilzen oder Mikroalgen, die einen Wertstoff, vorzugsweise ein Lipid, besonders bevorzugt omega-3-Fettsäuren, produzieren;
b) schonende Trocknung der erhaltenen Biomasse, wobei die schonende Trocknung das Überleiten von Gas in der Kreisgasfahrweise umfasst und wobei der Sauerstoffgehalt des Gases vorzugsweise weniger als 20 Gew.-%, insbesondere weniger als 15 Gew.-%, besonders bevorzugt 5 bis 13 Gew.-%, beträgt und zur Trocknung vorzugsweise das zuvor beschriebene Sprühgranulationsverfahren verwendet wird;
c) Vermischung der Biomasse und/oder daraus isolierter Wertstoffe, gegebenenfalls nach vorheriger Durchführung eines Zellaufschlussverfahrens, mit weiteren Nahrungs- oder Futtermittelinhaltsstoffen;
d) Herstellung des finalen Produkts durch ein Kompaktierungs- oder Extrusionsverfahren.

Ein erfindungsgemäß ganz besonders bevorzugtes Verfahren zur Herstellung eines Nahrungs- oder Futtermittels umfasst hierbei die folgenden Schritte:
a) Herstellung einer Biomasse, vorzugsweise durch Fermentierung von Pilzen oder Mikroalgen, vor allem von Netzschleimpilzen, die einen Wertstoff, vorzugsweise ein Lipid, besonders bevorzugt omega-3-Fettsäuren, produzieren;
b) schonende Trocknung der erhaltenen Biomasse auf einen Feuchtigkeitsgehalt von vorzugsweise weniger als 15 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%, wobei die schonende Trocknung das Überleiten von Gas in der Kreisgasfahrweise umfasst und wobei der Sauerstoffgehalt des Gases hierbei vorzugsweise weniger als 20 Gew.-%, insbesondere weniger als 15 Gew.-%, besonders bevorzugt 5 bis 13 Gew.-%, beträgt und zur Trocknung vorzugsweise das zuvor beschriebene Sprühgranulationsverfahren verwendet wird;
c) Überführung der Biomasse in eine Zellsuspension mit einem Feuchtigkeitsgehalt von mindestens 30 Gew.-%, vorzugsweise 30 bis 90 Gew.-%, besonders bevorzugt 40 bis 80 Gew.-%, insbesondere 50 bis 75 Gew.-%;
d) Zellaufschluss, vorzugsweise unter Anwendung eines Energieeintrags von maximal 50 kWh pro Tonne Suspension, vorzugsweise von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, vorzugsweise unter Verwendung eines Rotor-Stator-Systems;
e) Vermischung der aufgeschlossenen Zellen und/oder daraus isolierter Wertstoffe mit weiteren Nahrungs- oder Futtermittelinhaltsstoffen;
f) Herstellung des finalen Produkts durch ein Kompaktierungs- oder Extrusionsverfahren.

Erfindungsgemäß bevorzugte Verfahren zur Herstellung eines Nahrungs- oder Futtermittels zeichnen sich vorzugsweise dadurch aus, dass in keinem Verfahrensschritt ein höherer Energieeintrag auf die Biomasse erfolgt als 50 kWh pro Tonne Suspension. Vorzugsweise beträgt der Energieeintrag auf die Biomasse maximal 40 oder 35 kWh, insbesondere maximal 30 oder 25 kWh, besonders bevorzugt 20 oder 15 kWh, jeweils pro Tonne

Suspension. Auf diese Weise wird zusätzlich sichergestellt, dass der enthaltene Wertstoff möglichst wenig geschädigt wird.

Die aufgeschlossenen Zellen machen vorzugsweise 0,5-20 Gew.-%, insbesondere 1-10 Gew.-%, vorzugsweise 2-8 Gew.-% des Nahrungs- oder Futtermittels bzw. der zur Herstellung des Nahrungs- oder Futtermittels eingesetzten Zusammensetzung aus.

Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Als proteinhaltige Komponente, die zusätzlich Fette enthält, können beispielsweise eingesetzt werden Fischmehl, Krillmehl, Muschelmehl, Kalmarmehl oder Schrimpsschalen. Als fetthaltige Komponente kann alternativ auch Fischöl eingesetzt werden. Als fetthaltige Komponente kann auch ein Pflanzenöl eingesetzt werden, insbesondere Öl aus Sojabohnen, Rapssamen, Sonnenblumenkernen und Flachssamen. Als kohlenhydrathaltige Komponente kann beispielsweise Weizenmehl, Sonnenblumenmehl, Sojablumenmehl oder Getreidegluten eingesetzt werden.

Der Gesamtgehalt an Öl in dem Futtermittel - inklusive des Öls aus den öl-haltigen Zellen - beträgt vorzugsweise 15-50 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

Weiterer Gegenstand der vorliegenden Erfindung ist entsprechend auch ein Verfahren zum Züchten von Tieren, insbesondere Flossenfischen oder Crustaceen, vorzugsweise Lachs, bei welchem ein erfindungsgemäßes Futtermittel eingesetzt wird. Weiterer Gegenstand der vorliegenden Erfindung ist weiterhin ein Tier, insbesondere Flossenfisch oder Schalentier, das durch ein derartiges erfindungsgemäßes Verfahren erhältlich ist.

Gegenstand der vorliegenden Erfindung sind weiterhin Verfahren zur Gewinnung eines oxidationsempfindlichen Wertstoffs aus einer Biomasse, die einen erfindungsgemäßen Trocknungsschritt umfassen.

## Patentansprüche

1. Verfahren zur Trocknung einer Biomasse, die einen oxidationsempfindlichen Wertstoff enthält, **dadurch gekennzeichnet, dass** das Verfahren einen Trocknungsschritt umfasst, in welchem Trocknungsgas in Kreisgasfahrweise über die Biomasse geleitet wird.

2. Verfahren gemäß Anspruch 1, **dadurch gekennzeichnet, dass** es sich bei dem oxidationsempfindlichen Wertstoff um eine ungesättigte Fettsäure, vorzugsweise eine omega-3-Fettsäure oder omega-6-Fettsäure, handelt.

3. Verfahren nach Anspruch 1 oder 2, **dadurch gekennzeichnet, dass** es sich bei dem Trocknungsschritt um ein Wirbelschichtverfahren handelt, wobei die Wirbelschicht vorzugsweise eine Temperatur von 45 bis 95° C, insbesondere 45 bis 75 °C, besonders bevorzugt 50 bis 60 °C, aufweist.

4. Verfahren nach einem der vorhergehenden Ansprüche, **dadurch gekennzeichnet, dass** das Trocknungsgas einen Sauerstoff-Gehalt von weniger als 20 Gew.-%, vorzugsweise weniger als 15 Gew.-%, insbesondere 8 bis 13 Gew.-%, aufweist.

5. Verfahren nach einem der vorhergehenden Ansprüche, **dadurch gekennzeichnet, dass** die Biomasse Zellen des Taxons Labyrinthulomycetes, insbesondere solche der Familie der Thraustochytriaceae, besonders bevorzugt der Gattungen Thraustochytrium, Schizochytrium oder Ulkenia, enthält.

6. Verfahren nach einem der vorhergehenden Ansprüche, **dadurch gekennzeichnet, dass** die Biomasse in dem Trocknungsschritt in Form von Fermentationsbrühe mit einem Feststoffgehalt von 10 bis 50 Gew.-%, vorzugsweise 20 bis 40 Gew.-%, eingesetzt wird.

7. Verfahren nach einem der vorhergehenden Ansprüche, **dadurch gekennzeichnet, dass** die Biomasse in dem Trocknungsschritt in ein Granulat überführt wird.

8. Partikuläre Biomasse, erhältlich nach einem Verfahren nach einem der vorhergehenden Ansprüche.

9. Partikuläre Biomasse, die einen oxidationsempfindlichen Wertstoff enthält, **dadurch gekennzeichnet, dass** mindestens 80 Gew.-% der Partikel eine Korngröße von 100 bis 3000 Mikrometern aufweist und die partikuläre Biomasse vorzugsweise rieselfähig und staubarm ist.

10. Partikuläre Biomasse gemäß Anspruch 8 oder 9, **dadurch gekennzeichnet, dass** mindestens 90 Gew.-%, vorzugsweise mindestens 95 Gew.-%, der Partikel eine Korngröße von 100 bis 3500 Mikrometern, vorzugsweise 1500 bis 2500 Mikrometern, aufweisen.

11. Partikuläre Biomasse nach einem der vorhergehenden Ansprüche, **dadurch gekennzeichnet, dass** mindestens 50 Gew.-%, insbesondere mindestens 70 Gew.-%, vorzugsweise mindestens 90 Gew.-%, der Partikel im Wesentlichen sphärisch ausgebildet sind.

12. Partikuläre Biomasse nach einem der vorhergehenden Ansprüche, **dadurch gekennzeichnet, dass** es sich bei dem oxidationsempfindlichen Wertstoff um eine ungesättigte Fettsäure, vorzugsweise eine omega-3-Fettsäure, handelt.

13. Partikuläre Biomasse nach einem der vorhergehenden Ansprüche, **dadurch gekennzeichnet, dass** die Biomasse Zellen des Taxons Labyrinthulomycetes, insbesondere solche der Familie der Thraustochytriaceae, besonders bevorzugt der Gattungen Thraustochytrium, Schizochytrium oder Ulkenia, enthält.

14. Partikuläre Biomasse nach einem der vorhergehenden Ansprüche, **dadurch gekennzeichnet, dass** sie eine Schüttdichte von 400 bis 800 kg/m3, vorzugsweise 450 bis 700 kg/m3, aufweist.

15. Futter- oder Nahrungsmittel enthaltend eine partikuläre Biomasse nach einem der vorhergehenden Ansprüche sowie weitere Futtermittelinhaltsstoffe.
